# EUROPEAN PATENT SPECIFICATION

(11) **EP 2 134 836 B1**
(45) Date of publication and mention of the grant of the patent: **08.01.2014**
(21) Application number: 08719734.9
(22) Date of filing: 17.03.2008
(51) Int. Cl.: C12N 5/071, G01N 33/50

(54) **A COMBINED SCALABLE IN VITRO DIFFERENTIATION SYSTEM FOR HUMAN EMBRYONIC STEM CELLS FOR DIRECT ASSAY APPLICATION IN MULTIWELL PLATES**
KOMBINIERTES SKALIERBARES IN-VITRO-DIFFERENZIERUNGSSYSTEM FÜR MENSCHLICHEN EMBYRONALEN STAMMZELLEN FÜR DIE DIREKTTESTANWENDUNG IN MULTIWELL-PLATTEN
SYSTÈME COMBINÉ DE DIFFÉRENCIATION IN VITRO, POUVANT ÊTRE MIS À L'ÉCHELLE, POUR DES CELLULES SOUCHES EMBRYONNAIRES HUMAINES EN VUE D'UNE APPLICATION DE DOSAGE DIRECT DANS DES PLAQUES À MULTIPLES PUITS

(30) Priority: 16.03.2007 US 895201 P
(43) Date of publication of application: 23.12.2009
(60) Divisional application: 13159468.1
(73) Proprietor: Cellartis AB, 413 46 Göteborg (SE)
(72) Inventor: STREHL, Raimund, S-413 46 Goteborg (SE); ADLER, Sarah, 16225 Eberswalde (DE)
(74) Representative: Zacco Denmark A/S
(86) International application number: PCT/IB2008/050999
(87) International publication number: WO 2008/114204

(56) References cited:
- WO-A1-2005/049812
- WO-A1-2008/040532
- WO-A2-2005/005607
- ANONYMOUS: "TrypLE(TM) Select - Trypsin Replacement Enzyme" INTERNET ARTICLE, [Online] XP002412665 Retrieved from the Internet: URL:http://www.invitrogen.com/content/sfs/ brochures/GIBCO_CC19_V3.pdf> [retrieved on 2006-12-21]
- PENICK KITSIE J ET AL: "High-throughput aggregate culture system to assess the chondrogenic potential of mesenchymal stem cells." BIOTECHNIQUES NOV 2005, vol. 39, no. 5, November 2005 (2005-11), pages 687-691, XP002501113 ISSN: 0736-6205
- DAR AYELET ET AL: "Optimization of cardiac cell seeding and distribution in 3D porous alginate scaffolds." BIOTECHNOLOGY AND BIOENGINEERING 5 NOV 2002, vol. 80, no. 3, 5 November 2002 (2002-11-05), pages 305-312, XP003014569 ISSN: 0006-3592
- NG ELIZABETH S ET AL: "Forced aggregation of defined numbers of human embryonic stem cells into embryoid bodies fosters robust, reproducible hematopoietic differentiation." BLOOD 1 SEP 2005, vol. 106, no. 5, 1 September 2005 (2005-09-01), pages 1601-1603, XP002501115 ISSN: 0006-4971
- BURRIDGE PAUL W ET AL: "Improved human embryonic stem cell embryoid body homogeneity and cardiomyocyte differentiation from a novel V-96 plate aggregation system highlights interline variability." STEM CELLS (DAYTON, OHIO) APR 2007, vol. 25, no. 4, 21 December 2006 (2006-12-21), pages 929-938, XP002501116 ISSN: 1066-5099
- INVITROGEN: 'CyQUANT NF cell proliferation assay kit', [Online] 18 July 2006, Retrieved from the Internet: <URL:http://probes.invitrogen.com/media/pis /mp35006.pdf> [retrieved on 2011-03-29]
- BD BIOSCIENCES, [Online] 2006, BD biosciences Retrieved from the Internet: <URL:http://www.bdbiosciences.com/external_ files/dl/doc/manuals/live/web_enabled/35467 1_pug.pdf> [retrieved on 2011-12-09]
- SCAVO LOUIS M ET AL: "IGF-I regulates the differentiation of human mesenchymal stem cells into adipocytes", PEDIATRIC RESEARCH, vol. 51, no. 4 Part 2, April 2002 (2002-04), pages 112A-113A, & ANNUAL MEETING OF THE PEDIATRIC SOCIETIES'; BALTIMORE, MD, USA; MAY 04-07, 2002 ISSN: 0031-3998
- OGURA NAOMI ET AL: "Differentiation of the human mesenchymal stem cells derived from bone marrow and enhancement of cell attachment by fibronectin.", JOURNAL OF ORAL SCIENCE DEC 2004 LNKD- PUBMED:15901064, vol. 46, no. 4, December 2004 (2004-12), pages 207-213, ISSN: 1343-4934
- CHUNG YOUNG ET AL: "Human embryonic stem cell lines generated without embryo destruction", CELL STEM CELL, CELL PRESS, US, vol. 2, no. 2, 7 February 2008 (2008-02-07), pages 113-117, XP002604696, ISSN: 1934-5909, DOI: 10.1016/J.STEM.2007.12.013
- CHUNG YOUNG ET AL: "Supplemental Data: Human embryonic stem cell lines generated without embryo destruction", CELL STEM CELL, CELL PRESS, US, vol. 2, no. 2, 7 February 2008 (2008-02-07), pages 113-117, XP002608204, ISSN: 1934-5909, DOI: 10.1016/J.STEM.2007.12.013 [retrieved on 2008-01-10]

## Description

### Background

Human embryonic stem (hES) cells and cell types derived from them have the potential to revolutionize medicine in terms of their applicability *in vivo*, such as for cell therapies in order to replace damaged tissue, as well as *in vitro.* Concerning *in vitro* applications the hES cells and cells differentiated from them, such as different progenitor cell types, i.e. cell derivatives committed towards a specific cell lineage although still capable of proliferation (or self-renewal), provide an excellent tool in for instance the drug discovery process. Undifferentiated hES cells may be used e.g. for detecting substances having toxic effects on the developing embryo as well as for target evaluation and lead finding. Differentiated cells derived from hES cells, exemplified by but not limited to, cardiomyocytes or cardiac progenitor cells and dopaminergic neurons or neural progenitor cells may be used for target evaluation, hit and lead identification, and lead optimisation. In addition, hES cell-derived hepatocyte-like cells have a great potential for use in safety assessment and for ADMET (absorption, distribution, metabolism, elimination and toxicology) studies.

Of importance for the development of hES cell based assays is then the application of hES cells or cells derived from them in different multi-well plate formats, preferably in parallel, such as to generate monolayers for e.g. cytotoxicity testing as well as differentiating aggregates or microtissues for e.g. developmental toxicity testing. The processes for plate preparation need to be highly reproducible and scaleable to allow high throughput experimentation. Traditional hES cell handling for differentiation of the cells is often done with mechanical passaging and culture in small units which are methods not very suitable for the scale-up of these differentiation dependent assays in multi-well format culture plates thus making high throughput experimentation and analysis difficult. Conventional feeder-free seeding of hES cells usually results in variable attachment and leads to increased variability between individual wells and plates. Differentiation of hES cells to obtain cell types of all three germlayers, endoderm (giving rise to e.g. the liver, pancreas, lungs, and gut), mesoderm (giving rise to e.g. muscle, tendon, bone and cartilage) and ectoderm (giving rise to e.g. skin and the three neural lineages neurons, astrocytes, and oligodendrocytes) is ordinarily achieved via embryoid body (EB) formation which requires suspension culture and subsequent plating of three-dimensional hES cell aggregates. EB culture involves several tedious steps and requires careful manual handling during e.g. culture medium renewal. Efforts have previously been made to automate hES cell differentiation as EBs from small hES cell clumps in for instance horizontally rotated, fluid-filled culture vessels or bioreactors, (WO2004050826, Technion). No matter the scalability of the EB formation method, multiple steps and transfers of the cell material to other readable formats, suitable for assays, such as multi-well format plates are needed.

In W02005049812 (Elefanty et. al.) and in (Ng et. al. 2005) is presented a method for obtaining blood cells from hES cells (hematopoiesis) via EB formation by spinning of hES cells. The blood cells are plated, i.e. transferred to another culture plate. The EBs are thereafter plated. No indication is given to a method or a system for scalable hES cell differentiation enabling direct assay use, in which assays on the cell material is performed in the very same plate without transfer of the material, and preferably without any washing steps (homogeneous screening).

Similar techniques are disclosed in Penick Kitsie et al. 2005, using mesenchymal stem cell aggregation to assess chondrogenic potential and also in WO2005/005607, where partially differentiated pancreatic-like cells are seeded onto 3D scaffolds. No indication is given in any of this background art of a method or a system for scalable hES cell differentiation enabling direct assay use, in which assays on the cell material is performed in the very same plate without transfer of the material, and preferably without any washing (homogeneous screening). Multiwell plates have been used previously to generate differentiated cell types from hES cells (Burridge et al. 2006).

Such a scalable and direct differentiation method is presented herein, allowing facilitated analysis on gene as well as on protein level. If used in combination with or following a system allowing large culture quantities of undifferentiated hES cells, the herein presented system can provide large amounts of differentiated cells from hES cells in multiwell formats compatible with HTS/MTS assay use. Moreover, the herein presented system is less labour intensive than known techniques and, furthermore, allows automation and robotic handling.

### Summary

The inventors of the instant application have developed differentiation systems for human embryonic stem (hES) cells.

In one aspect, the instant invention provides combined differentiation and assay methods for human embryonic stem (hES) cells, wherein the combined method is performed in the very same plate without any transfer of the cells, the method comprising the steps:
(i) dissociation of the hES cells into single cells, clusters or a mixture thereof;
(ii) seeding of the dissociated hES cells into one or more wells of one or more multi-well format plates;
(iii) application of a centrifugal force to the seeded hES cells for enhanced coalescence; and
(iv) differentiating the hES cells into progenitor cells or fully differentiated cells of the endodermal lineage and assaying the cells without replating;
wherein no replating of cells takes place in steps (ii) to (iv).

The hES cells after differentiation are progenitor cells or fully differentiated cells of the endodermal lineage.

In one embodiment, the hES cells after differentiation are hepatoblast-like cells.

In another embodiment, the hES cells after differentiation are a mixed population of more than one cell type.

In another embodiment, the hES cells after differentiation are an essentially pure population.

The differentiation and assay use take place without any transfer of the cells between different plates.

The differentiation and assay analysis take place in the same multi-well plate.

In another embodiment, the cell dissociation agent allows dissociation into single cells or to a cluster size of between 1 and 20 cells.

In another embodiment, the cell dissociation agent allows dissociation to cluster sizes of between 20 and 500 cells.

In another embodiment, the cell dissociation agent is a mechanical tool, such as a sharp micro capillary allowing dissociation of hES cells and cells derived from hES cells into clusters.

In another embodiment, the multi-well format plates are plates with between 12 and 1536 wells, such as between 48 and 384, such as 96 wells.

In another embodiment, the multi-well format plates in are conical-bottom or round-bottom bottom plates to allow enhanced coalescence to microtissue.

In another embodiment, the multi-well format plates are flat-bottom plates to allow enhanced coalescence to cell monolayers.

In another embodiment, the differentiation and assay methods further comprise one or more cell strainers, e.g., for the even distribution of the hES cells or hES-derived cells between individual wells of the multi-well format plates.

In another embodiment, the differentiation and assay methods further comprise the centrifugal force being applied by use of a centrifuge and rotor compatible with the multi-well format plate.

In another embodiment, the dissociation agents are selected from a group consisting of Trypsin/EDTA, TrypLE-Select, Collagenase IV and Dispase

In another embodiment, the culture media for incubation of the cells contains a serum component, selected from a group consisting of FBS, serum replacement, human serum, albumin, insulin, and transferin. In specific embodiments, these components are present at a concentration of 0.5-50%, such as 5-30% or such as 10-20%.

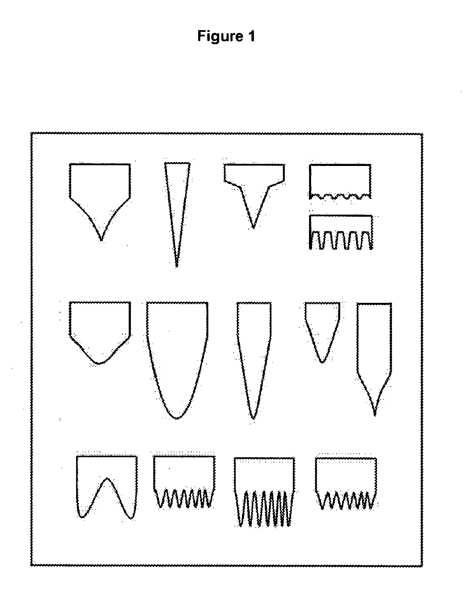

In another embodiment, the differentiation and assay methods further comprise one or more multi-well format plates are selected from a group of shapes as set forth in figure 1.

In another embodiment, the differentiation and assay methods further comprise centrifugal force for enhanced coalescence of the cells such 5 - 10 000 G, such as 100-1000 G, such as 400 G.

In another embodiment, the differentiation and assay methods further comprise the cells are cultured and/or incubated for 1 hour-90 days, such as 1-30 days, such as 4-10 days, such as 7 days.

In another embodiment, the number of cells at seeding ranges from 500 - 500 000 cells, such as 1000- 1000 000 cells, such as 10 000 cells.

In one embodiment, the differentiation and assay method as disclosed above comprises:
i) dissociating the hES cells with one or more dissociation agents;
ii) seeding of the hES cells into one or more individual wells in one or more multi-well format plates at a density of 1000 -1000 000 cells per well;
iii) applying a centrifugal force, such as 100-1000 G, for enhanced coalescence of the hES cells;
iv) incubating the hES cells in one or more culture media containing a serum component, selected from a group including, but not limited to, FBS, serum replacement, human serum, albumin, insulin, and transferin, at a concentration of such as 5-30%, to differentiate them into progenitor cells or fully differentiated cells of the endodermal lineage and assaying the cells without replating.

In one embodiment, a survival promoting substance is added during step (i) and/or (ii)

In one embodiment, the cells are strained.

In specific embodiments, the invention provides methods of producing cells to test embryo toxicity, to test teratocgenecity, for use in drug discovery and/or for safety assessment and toxicity studies, for preparing microtissues and monolayers from the very same cell suspension of hES cells for the performing of a comparative analysis, for performing developmental toxicity assays and differentiation assays, for preparing cells for detecting or measuring cell differentiation.

In one embodiment, the cell differentiation is measured by measuring gene or protein levels, e.g., by immuno-cytochemistry.

In specific embodiments the invention provides assays based on genetically engineered cells, such as an hES cell line carrying reporters for selected marker genes, for assays based on quantitative PCR, for running an assay measuring the levels of a substance or compounds in the cell culture medium, for assays to detect human toxicity, and for running an assay to detect or measure cytotoxicity.

In another aspect, the invention further provides the use of a kit in a method according to the invention, the kit comprises one or more dissociation agents, selected from Trypsin/EDTA, Collagenase IV and Dispase. In a related embodiment, the kit further comprises a rotor for plate centrifugation.

In another embodiment, the kit further comprises additional tools for in vitro assay performance, e.g., reagents for PCR, immunochemistry reagents for the detection or quantification of pluripotency, or immunochemistry reagents for the detection or quantification of germ layer specific differentiation.

In another embodiment, the kit further comprises positive control substances, negative control substances or additional cell types for use as control populations.

Differentiation systems for human embryonic stem (hES) cells may comprise:
(i) one or more dissociation agents for dissociation of the hBS colonies;
(ii) one or more culture media for incubation of the cells:
(iii) one or more multi-well format plates for seeding of the cells into one or more individual wells;
(iv) a centrifugal force for enhanced coalescence of the cells;
(v) wherein the cells in the multi-well format plates are directly applicable for assays without replating.

The system may further comprise one or more cell strainers for the even distribution of the cells between individual wells of the multi-well format plates.

Differentiation systems for cells derived from hES cells may comprise the same components, i.e.:
(i) one or more dissociation agents for dissociation of the cells;
(ii) one or more culture media for incubation of the cells:
(iii) one or more multi-well format plates for seeding of the cells into one or more individual wells;
(iv) a centrifugal force for enhanced coalescence of the cells;
(v) wherein the cells in the multi-well format plates are directly applicable for assays without replating.

The system may further comprise one or more cell strainers for the even distribution of the cells between individual wells of the multi-well format plates.

A feature of the present invention is that the cells derived from hES cells in the differentiation system are directly applicable for assays, in large variety of assay units, such as different multiwell plates. The herein presented system is therefore a major improvement compared to the prior art, since the cells are differentiated in the same format as they are further being subject to analysis in. The starting cell material can be homogenously distributed across a variety of different plates, for use and can be cultured attached, semi attached or in suspension.

### Brief Description of the Drawings

*Figure 1* shows examples of well geometrics for adjustment of the sterical relationship between cells in the invention.

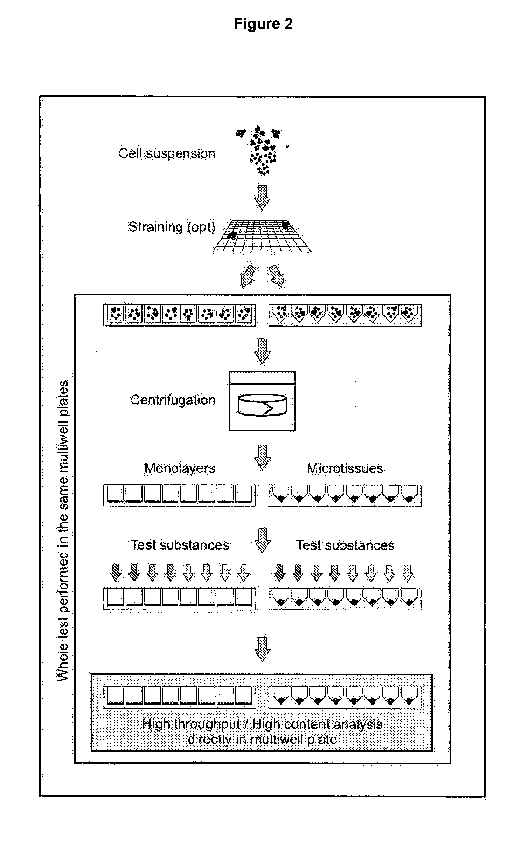

*Figure 2* shows a schematic chart over the invention starting out with a cell suspension that goes via an optional straining step and centrifugation into the multi-well format plates, in which both differentiation and assay use can take place without additional transfer steps of the cell material.
*Figure 3* shows hES cells differentiated in a monolayer in a flat-bottom 96-well plate for 12 days.
*Figure 4* shows microtissue differentiated in a conical-bottom 96-well plate for 12 days.
Figure *5* shows large microtissue differentiated in a round-bottom polypropylene 96-well plate for 30 days, a) scale bar = 1000 um b) scale bar = 200 um
*Figure 6* shows contracting microtissue (in circle) differentiated in a conical-bottom 96-well plate for 12 days, i.e. the herein presented system also allows formation of contracting areas indicating the presence of cardiomyocytes-like cells.
*Figure 7* shows cytoxic values for valproic acid run on hES cells with a resazurin assay, as described in Reference Example 5.
*Figure 8* shows time dependent gene expression of microtissues derived from cell line SA002 at day 0, 7 and 14. Cells were differentiated in DMEM-F12 supplemented with 1% penicillin-streptomycin, 1% Glutamax, 0.5 mmol/l β-mercaptoethanol, 1% non-essential amino acids and 20% Knockout serum replacement in conical 96-well plates. Experiments were performed in three independent runs (n=3). Standard error bars are shown.
*Figure 9* shows the percentage of contracting areas formed depending on the cell number per well and time.

### Detailed Description

### Definitions and abbreviations

As used herein, the term "assay" is intended to mean a cell-based analysis in order to detect or measure a reaction to at least one concentration of one or more substances.

By the term "cell strainer" is intended to mean a mesh or grid with a pore size making it suitable for filtrating cell suspensions allowing cell clusters up to a certain diameter to pass through.

As used herein, the terms "direct assay application" and corresponding "directly applicable for assays" are intended to mean that the assay can take place without any transfer to or plating of the cells in new culture plates or multi-well format plates.

As used herein, the term "enhanced coalescence" is intended to mean increased attachment of cells either to each other or to a surface by the application of an external force.

As used herein, the term "essentially pure population" is intended to mean a cell population, wherein at least 80%, such as at least, 90%, at least 95%, such as 98% are cells of the same type.

As used herein, the term "cells derived from hES cells" is intended to mean cells selected from a group of one or more cell types exemplified by but not limited to: progenitor cells of endodermal, mesodermal, and ectodermal origin, as well as fully differentiated cells, such as, e.g., hepatocytes, beta-cells, cardiomyocytes, chondrocytes, osteocytes, keratinocytes, neurons, oligodendrocytes or astrocytes.

By the terms "feeder cells" or "feeders" are intended to mean cells of one type that are co-cultured with cells of another type, to provide an environment in which the cells of the second type can grow. The feeder cells may optionally be from a different species as the cells they are supporting. The feeder cells may typically be inactivated when being co-cultured with other cells by irradiation or treatment with an anti-mitotic agent such as mitomycin c, to prevent them from outgrowing the cells they are supporting. Without limiting the foregoing, one specific such feeder cell type may be a human feeder, such as a human skin fibroblast, here denoted as hFF. Another feeder cell type may be mouse embryonic fibroblasts (mEF).

As used herein, the term "microtissue" is intended to mean attached as well as non-attached three dimensional structures formed from the cells *in vitro.* Microtissues consist of cells and their extracellular matrix.

As used herein, the term "monolayer" is intended to mean a two-dimensional culture, as exemplified but not limited to the culture on the bottom of a flat well in multi-well plate.

The interpretation of the term "substance" is not intended to be limited to therapeutic agents (or potential therapeutic agents), or agents with documented toxicity effects such as neurotoxins, hepatic toxins, toxins of hematopoietic cells, myotoxins, carcinogens, teratogens, or toxins to one or more reproductive organs. The term substances may further be used to denote chemical compositions such as agricultural chemicals, e.g. pesticides, fungicides, fertilizers, or components used in cosmetics and as food additives.

### Detailed description of the invention

A feature of the present invention is that the cells derived from hES cells in the differentiation system are directly applicable for assays, in large variety of assay units, such as different multiwell plates. The herein presented system is therefore a major improvement compared to the prior art, since the cells are differentiated in the same format as they are further being subject to analysis in. No replating or further handling is necessary.

The starting cell material can be homogenously distributed across a variety of different plates, for use and can be cultured attached, semi attached or in suspension.

### Cells

The starting material of the present invention is undifferentiated hES cells. The system is of course applicable for all types of pluripotent or multipotent stem cells. Other pluripotent stem cells may be obtained from different sources, such as e.g. placenta and amniotic fluids. Multipotent stem cells may be obtained from different sources such as the adult body, and from differentiation of pluripotent stem cells *in vitro.*

Typical characteristics of undifferentiated hES cells recommended as starting material are the following: positive for alkaline phosphatase, SSEA-3, SSEA-4, TRA 1-60, TRA 1-81, OCT-4, negative for SSEA-1, telomerase activity, and pluripotency *in vitro* and *in vivo* (the latter shown by teratomas formation in immuno-deficient mice). The hES cells providing the starting material may be routine-cultured on mEF cells with mechanical passaging techniques using a sharp microcapillary. In addition, hES cells passaged enzymatically and cultured either feeder-free or on mEF or other feeder cell types, such as hFF may be used.

For transfer into the present system the cells may be removed from their normal culture system by use of enzymes, chelators, or combinations thereof and thereafter dissociated into a suspension of clusters, single cells or a mixture of single cells and clusters, enzymatically or mechanically. The cell colonies may also be detached mechanically from their previous culture system and followed by optimal subsequently enzymatically dissociated before being put into the herein presented culture system.

Differentiation in the herein presented system may take place between 1 and 28 days, such as between 2 and 21 days, such as between 7 and 14 days. In one specific embodiment of the present invention differentiation takes place 12 days. The incubation with a substance, the effect of which is subject to study in the assay, may be carried out while the cells are differentiating in the herein presented system or after that the cells have differentiated for a defined amount of time.

Significant for the present invention is that the differentiation system allows differentiation and the assaying takes place in the same plate without any transfer of the cells between different plates. In still further embodiments the herein presented system may allow a completely homogenous assay use, eliminating also washing steps. The system allows homogenous distribution of cells across different multiwell plate types for assay use such as plates with different well geometry or surface charcteristics. Cells can be maintained attached, semi-attached and suspended depending on the specific application, which will significantly facilitate medium change and automation of the assay.

### Dissociation agents

The cell dissociation agent in item (i) may allow dissociation into single cells or to a cluster size of between 1 and 20 cells, such as between 2 and 10 cells. Of course also larger cluster sizes may be used in the present invention.

For dissociation to single cells a chelator, such as EDTA or one of the following enzymes may preferably be used: Trypsin, TrypLESelect™, Accutase, or AccuMax. In addition a combination of chelator and enzyme may be applied. The dissociation agent may preferably be dissolved in calcium and magnesium free PBS to facilitate dissociation to small clusters and single cells.

To enhance the survival of the used cells a survival promoting substance can be added, (eg. growth factor, inhibitors of cellular pathways involved in cell death and apoptosis, conditioned media.

For larger cluster sizes, such as from between 20 to 500 cells, such as from between 40 and 400 cells, a different dissociation agent or a different mixture of dissociation agents are preferably used, such as e.g. Collagenase IV or Dispase.

Enzymatic dissociation to single cell suspension or to larger clusters may be supported by mechanical force.

The dissociation agent may alternatively be only a mechanical force, such as by using a mechanical tool, such as a pipette or a sharpened micro capillary to detach the cells. The removed cells may then be transferred as large clusters of up to 50 000 cells e.g. whole cell colonies or further dissociated mechanically by triturating with e.g. a pipette.

Cell numbers per well can range from 1 to 5 000 000 cells/well, such as 10 000 to 50 000, such as 5 000 to 100 000, depending on application, well size and geometry. For the 96 well plate format the cell number is preferably 10 000 - 20 000 cells/well for a flat well and 50 000 - 100 000 cells/well for conical wells.

### Culture media

The culture medium in item (ii) of the herein presented system may be any suitable base medium, supplemented with nutrients. Examples of suitable such media are VitroHES™ (Vitrolife) and serum-based media with either human serum or animal serum or serum-free medium of completely defined composition or with a serum replacement. The medium can be chosen to allow spontaneous differentiation of the hES cells into multiple cells types to recapitulate early human embryonic development in vitro. The medium is alternatively chosen to be suitable for the respective cell type to be generated, i.e. the desired differentiation pathway. The medium may also be supplemented with human recombinant growth factors, differentiation factors and driving agents and/or potential other additives, such as e.g. activin, bFGF, Epithelial Growth Factor, Hepatocyte Growth Factor (HGF) and Oncostatin M., for endodermal differentiation, BMPs (bone morphogenic proteins), EGF and FGF families, B-27, 5-aza deoxycytidine, DMSO, retinoic acid, ASA and ascorbic acid for mesodermal differentiation and ascorbic acid or cyclic AMP for neural differentiation. The medium may further be supplemented with agents which influence the medium's viscosity such as matrigel, polyvinyl alcohol, methylcellulose, agar, collagen, hyaluronic acid or alginate in order to influence the cells mobility and sterical arrangement.

In a preferred embodiment of the present invention the following medium composition is used: Knock Out DMEM supplemented with 20% Foetal bovine Serum (FBS), 1% (v/v) penicillin-streptomycin, 1% (v/v) Glutamax, 0.5 mmol/I β-mercaptoethanol and 1 % (v/v) non-essential amino acids (all Invitrogen, Carlsbad, California).

### Multi-well format plates

The multi-well-format plates in item (iii) of the differentiation system may be multi-well format plates with between 6 and 1536 wells, such as between 12, 48 and 384, such as 96 wells.

The plates may further be of different sorts of plastic, such as e.g. tissue culture treated polystyrene, or Primaria™ plastic or even coated with a suitable protein or extracellular matrix component, such as e.g. matrigel, laminin, or gelatin or different combinations thereof. One other coating option is to have a suitable feeder cell type, such as e.g. mEFs or hFFs growing in the plates of the present invention and thereafter treat the feeder cells with for instance a detergent to produce a feeder cell deposited matrix or even use live but preferably mitotically inactivated feeder cells as a live-coating matrix. The effect of the present live feeder cells should then of course be carefully analysed in order to make sure they do not interfere with the assay read-outs.

After initial attachment by centrifugation, the cells may be overlaid with further suitable proteins or with additional cell types. Centrifugation can be repeated to form multilayer structures.

The wells can also contain a suitable scaffold in material such as PLGA (poly(lactic-co-glycolic acid) Scaffold, poly (D,L-lactic acid) (PLDA) or hydroxyapatite, with a semi-porous, solid, open pore or sponge conformation.

The multi-well format plates may in addition be of attachment repellent material such as molded silicone or polypropylene or have attachment-repellent surface treatment such as ultra-low attachment surface (Corning) or a repellent coating such as silane to promote cell-cell attachment over cell-plate attachment during the formation of microtissues.

In a preferred embodiment of the present invention non-coated tissue culture plastic 96-well plates are used. In another preferred embodiment of the present invention gelatin coated 96-well plates are used.

The multi-well format plates may further be of certain geometry, such as conical-bottom or round-bottom bottom plates in order to allow enhanced coalescence to microtissue or flat-bottom plates to allow enhanced coalescence to cell monolayers.

Typical features of the microtissue formed by the enhanced coalescence, is that they at day 0, i.e. directly after centrifugation and depending on well size, geometry and the number of cells seeded per well, may have a diameter of between 10µm and 6000 µm.

In another preferred embodiment of the present invention undifferentiated hES cells are seeded in commercially available flat-bottom wells to generate monolayers comprising a mixed population of cells derived from hES cells.

In still another embodiment of the present invention the undifferentiated hES cells are seeded in parallel both into round- or conical-bottom plates to generate microtissues and into flat-bottom multi-well format plates to generate monolayers, a procedure that can significantly improve a combined assay approach, such as a combination of a cytotoxicity assay and an *in vitro* developmental assay.

In a preferred embodiment both plates described above can be used in combination as part of a testing strategy to generate human relevant data for developmental toxicology in increased throughput while avoiding further replating or handling of cells.

Still further optimisation concerning the multi-well format plates is possible by application of plates with a custom designed geometry to potentially influence cell aggregation, such as e.g. deep steep walled half-elliptical wells, shallow steep walled conical wells or geometric as described in figure 1, to adjust sterical relationship between cells.

### Cell strainer:

The herein presented system may in addition comprise one or more cell strainers to retain larger clusters and for the even distribution of the hES cells between individual wells of the multi-well format plates. Suitable material for such cell strainers are fiber, fabric, plastic, and nylon. The grid size is preferably of a defined size of between 20 and 200 µm.

### Robot:

The production and manipulation of hES cells is currently being scaled up using novel culture systems for bulk culture, therefore large amounts of hES cells will become available for the application in *in vitro* assays. The preparation, treatment and analysis of cells derived from hES cells in multi-well format plates according to our system and related method does not require manual selection or micromanipulation and can therefore be scaled up and automated using robotization. Suitable robots could be based on XYZ dispensing heads which allow pipetting to and from culture vessels such as liquid handling stations or could be based on a robotic arm which mimics the movements of a human being during culture vessel and pipette handling. Within the robotic system environmental parameters such as e.g. temperature, nutrient supply, pH, pressure, shear forces and oxygen should be maintained within optimal limits. The possibility to create attached or semi-attached microtissues significantly improves applicability to robotization as manipulation such as medium change are possible without the risk of accidentally removing the microtissue.

### Method:

The present invention relates to a differentiation method for direct assay application of cells derived from hES cells, comprising the following steps:
(i) dissociation of the hES cells into single cells, clusters or a mixture thereof;
(ii) seeding of the dissociated hES cells into one or more wells of one or more multi-well format plates;
(iii) application of a centrifugal force to the seeded hES cells for enhanced coalescence;
(iv) differentiating the hES cells into progenitor cells or fully differentiated cells of the endodermal lineage and assaying the cells without replating; wherein no replating of cells takes place in steps (ii) to (iv).

The cells may in step (i) be dissociated into single cells or cell clusters by use of the components as described above.

In addition the cells may be strained by use of a cell strainer as also described above.

The centrifugal force may be applied in step (iii) by centrifuging the multi-well format plates once or by repeated centrifugation during the incubation in step (iv). The centrifugation process can be applied continuously. The centrifugation may further be performed at a temperature of between 4 and 37 °C, such 20 °C. The centrifugation may further be performed in ambient atmosphere or in defined gas atmosphere as well as defined humidity. The time for centrifugation may be between 1 and 240 min, such as between 3 and 20 minutes, such for 5 minutes.

In a preferred embodiment of the present invention the multi-well format plates are centrifuged once at 400-1500 g, 5-10 min in room temperature (20 °C) in ambient atmosphere, but the parameters may of course vary with the cell type and application.

The application of a centrifugal force to the seeded hES cells, will lead to a more homogeneous attachment of cells as a monolayer in flat-bottom wells, whereas it will lead to a more homogeneous formation of microtissues in conical or round bottom wells. The defined centrifugation step (iii) promotes cell-matrix adhesion during monolayer formation as well as cell-cell aggregation during microtissue formation.

The herein claimed differentiation method can be further refined by custom formed wells in the future. The formed microtissues can grow semi-attached to the plate and may therefore allow differentiation of hES cells directly in the multi-well format plates without a subsequent re-plating step. In contrast to suspension culture using embryoid bodies the handling of slightly attached microtissues has significant handling advantages, such as e.g. medium renewal can be performed in an automated fashion without the risk of aspiring the suspended cell clusters.

The incubation in step (iv) may be incubation with a substance for a longer period of time, such as from the actual seeding in step (ii) until running the actual analysis after between 1 and 56 days, such as after 12 days. The incubation may in addition be acute, i.e. for less than one day, such as for less than 12 hours, such as for as little as 1 minute.

During the incubation one or more medium changes may be performed in order to maintain medium components and the substance for testing at as stable concentration levels as possible.

In the present invention the incubation takes place while the cells are differentiating.

In a preferred embodiment the incubation is initiated one day after that the cells were seeded and centrifuged.

As described above, the preparation, treatment and analysis of cells derived from hES cells in multi-well assay plates according to our system and related method does not require manual selection or micromanipulation and can therefore be scaled up and automated using robotization.

### Use:

Further described is the use of a differentiation system as described herein for embryo toxicity and/or teratogenecity and/or developmental toxicity studies.

Also described is the use of a differentiation system as described herein, to produce differentiated tissues for the use, in drug discovery and/or for safety assessment or toxicity studies.

The system as described herein may also be used for screening of e.g. novel monoclonal antibodies as well as for detecting novel substances with medicinal effects or detecting novel effects of known substances.

The herein presented system may also be used for preparing microtissues and monolayers from the very same cell suspension of hES cells or cells derived from hES cells for the performing of a comparative analysis, i.e. running for instance one cytotoxicity analysis on microtissues in parallel with an antibody-based HCS assay on monolayers.

The herein presented differentiation system may be used for performing developmental toxicity assays and differentiation assays, such as for assays to detect or measure cell differentiation on e.g. gene or protein level. Examples of suitable endpoints on which the developmental toxicity assay may be based are: immuno-cytochemistry, fluorescence reporters in genetically engineered cells or quantitative PCR.

The toxicity test may be started by adding the toxicity solution to the test wells and then perform medium changes with a suitable frequency throughout the assay period. Several toxic substances may be tested in suitable dilution series and preferably a known toxicant is used as control substance. For the antibody based assay approach the cells may need fixation and potentially permeabilisation (if to detect or measure non-surface bound antigens) as well as washing steps in between the incubations with primary and secondary antibodies. Thereafter the cells may be analyzed using e.g. image analysis techniques, such as an automated cell imager like the IN Cell Analyzer (IN Cell Analyzer 1000, GE Healthcare, Uppsala, Sweden) that comprises an automated Nikon™ microscope, high-resolution CCD camera, xenon lamp-based illumination, filter wheel based wavelength control, and laser based auto focus. Employing image analysis software packages, the instrument can acquire population-averaged and individual cell data from a wide variety of live-cell and fixed-cell applications.

Still one additional assay approach could be to use membrane binding antibodies to perform high content screening (HCS) immunohistochemistry on living cells. Such antibodies may be directly conjugated to a fluorescencent marker whereby several washing steps can be eliminated also leading to a simplified and more homogeneous assay protocol. RT-qPCR using selected genes using a set of genes (table 1) or using low density arrays in high trough put RT-qPCR equipment such as AB1 2900 HT.

In parallel genetically engineered hES cells containing a marker gene, such as the GFP gene driven under a suitable undifferentiated hES cell specific promoter, such as the OCT-4 promoter or under a germ layer specific promoter, such as sox-1, AFP and/or HNF3-β, may be applied virtually in the same manner, although a much more simplified procedure may be applied, in which fixation, permeabilisation and washing steps may be eliminated, which is one big advantage with the latter approach. In addition, the reporter-gene approach has the advantage of enabling reading over time, i.e. not only giving a snap-shot of the cells at a specific moment (the moment of cell fixation). Continuous kinetic analysis over a time frame of minutes to weeks becomes possible.

Further described is the use of the differentiation system for assays measuring the levels of a substance or compounds in the cell culture medium. Examples of compounds present in the cell culture medium are various biomarkers such as cell metabolites or other molecules secreted by the cells, or taken up consumed by the cells.

Also described is the use of the differentiation system for assays to detect human toxicity, such as cytotoxicity. Methods to measure cytotoxicity can be selected from a group including but not limited to colorometry, fluorometry, luminescence, absorbance and radiometry, further exemplified by resazurin conversion, and ATP content analysis. Such a cytotoxicity test may be initiated by adding toxicity solution to the test wells and during the incubation period change the medium a few times. The multi-well format plates containing the hES cells or cells derived from hES cells may then be analysed measuring the reduction of Resazurin (Sigma, Stockholm, Sweden, CAS 62758-13-8) to the fluorescent Resofurin, which may be detected using a multi-detection reader (Fluostar Optima, BMG Labtech, Offenburg, Germany) measuring fluorescence, at the wave lengths 530 nm (excitation) and 590 nm (emission). The assay then generates values on cytotoxicity of the substance tested which can be illustrated in a graph.

### Kit:

In still further aspects the present invention relates to the use of a kit in the method of the invention, the kit comprises:
i. one or more dissociation agents, selected from trypsin/EDTA, collagenase IV and dispase.
ii. one or more types of multi-well format plates;
iii. (optional) cell strainer;
iv. (optional) a user manual for the preparation of the herein claimed culture system.

Such a kit may further comprise a rotor for plate centrifugation.

Still suitable components for such a kit for use according to the present invention may be tools for *in vitro* assay performance, such as e.g. PCR or immunochemistry markers for the detection or quantification of pluripotency or for the detection or quantification of germ layer specific differentiation cytotox markers apoptosis and stress markers and (optional) a suitable user assay manual.

A kit for use according to present invention may in still further aspects comprise positive and negative control substances as well as additional cell types for use as control populations.

### Examples

### Reference Example 1

### Starting material

### Undifferentiated hBS cells

The starting material is pluripotent undifferentiated hES cells, such as undifferentiated hES cell lines. Such material can be obtained from Cellartis AB and is also available through the NIH stem cell registry http://stemcells.nih.gov/research/registry/. Cellartis AB has two hES cell lines (SA001 and SA002) and one subclone of SA002 (SA002.5) available through the NIH. All the hES cell lines used are approved and registered by the UK Stem Cell Bank Steering Committee and SA001, SA002 and SA002.5 are approved by MEXT (Japan). Characteristics of the hES cells recommended as starting material are the following: positive for alkaline phosphatase, SSEA-3, SSEA-4, TRA 1-60, TRA 1-81, OCT-4, negative for SSEA-1, telomerase activity, and pluripotency *in vitro* and *in vivo* (the latter shown by teratomas formation in immuno-deficient mice). *(Methods and protocols as previously shown,* Heins et al, WO2003055992*.)* The hES cells providing the starting material may be routine-cultured on mEF cells with mechanical passaging techniques using a sharp microcapillary. In addition hES cells passaged enzymatically and cultured either feeder-free or on mEF or other feeder cell types, such as hFF may be used.

hES cells cultured on mitotically inactivated human feeder cells (hFF, ATCC CRL-2429) at a density of around 70.000 hFF cells/cm² and passaged enzymatically (TrypLE™Select, Invitrogen) were instead after enzymatic dissociation into single cells seeded onto on a feeder density of 20.000 cells/cm². After 8 to 10 days in culture the culture medium was removed from the dish and the cells were washed once with PBS (Gibco Invitrogen). Following, the cells were incubated with 1 mg/ml Collagenase IV (Invitrogen) solution in DMEM/F12 (Gibco Invitrogen) at 37°C for 30 minutes until ca. 60 % of the colonies were detached. The Collagenase solution including the cell colonies was taken up with a 5 ml pipette (BD, Stockholm, Sweden) and transferred into a 15 ml tube (BD Biosciences). After ca. 5 min the colonies had sunk to the bottom of the tube and the supernatant including some single feeder cells was removed. Fresh culture medium was given into the tube and the colonies were dissociated into small aggregates by pipetting up and down with a 1000 µl automatic pipette (Thermo) before being seeded into the wells of the multi-well format plates as described below.

### Reference Example 2

### Enhanced coalescence in conical- versus flat-bottom wells

### Flat-bottom wells - monolayer formation:

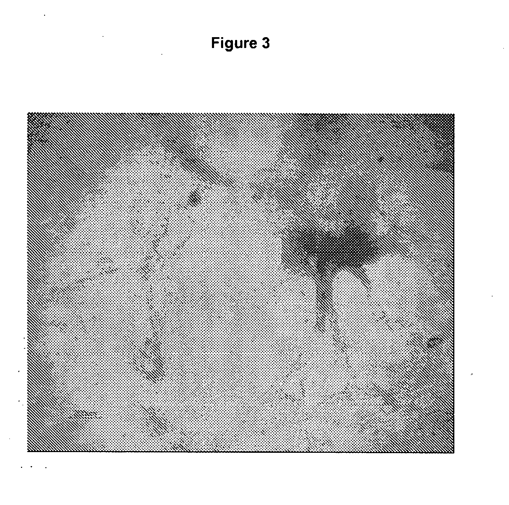

hES cells (SA002,5, p44) were dissociated into small cell aggregates and seeded into a 0,1% gelatine (Sigma, Stockholm, Sweden) coated 96-well flat-bottom plates (Nunc, Kamstrupvej, Denmark) in 200µl cell culture medium containing Knock Out DMEM supplemented with 20% FBS, 1% penicillin-streptomycin, 1% Glutamax, 0.5 mmol/l β-mercaptoethanol and 1% non-essential amino acids (all from Invitrogen, Carlsbad, California) in a concentration of ca. 10000 cells per well. Following the plates were centrifugated at 400g for 5 minutes in a centrifuge (Eppendorf). The hES cells were let to differentiate for 10 days with medium change every second day. Photos were taken at day 10 using a light microscope (Leica Microsystems AB, Sollentuna, Sweden). The hES cell-derived monolayers showed high levels of cellular proliferation and morphologies of primitive tissues. (*See* *figure 3**.)*

### Conical wells - microtissue formation:

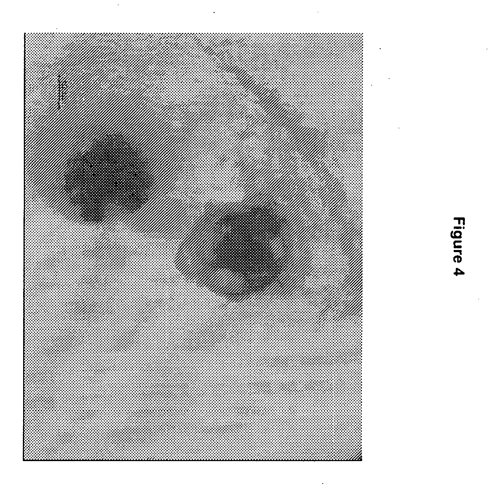

hES cells (SA002,5, p38) were dissociated into small cell clusters of between 50 and 100 cells per cluster and seeded into 96-well conical-bottom non-tissue culture treated plates (Nunc, Kamstrupvej, Denmark) in 200µl cell culture medium containing Knock Out DMEM supplemented with 20% FBS, 1% penicillin-streptomycin, 1% Glutamax, 0.5 mmol/l β-mercaptoethanol and 1% non-essential amino acids (all from Invitrogen, Carlsbad, California) in a concentration of ca. 10000 cells per well. Following the plates were centrifugated at 400g for 5 minutes in a centrifuge (Eppendorf). The hES cells were let to differentiate for 12 days with medium change every second day. Photos were taken at day 12 of differentiation using a light microscope (Leica Microsystems AB, Sollentuna, Sweden). At this time point the cells have formed microtissue with a diameter of approximately 500 to 1000 µm. *(See* *figure 4**.)*

### Formation of large microtissues for scalable culture:

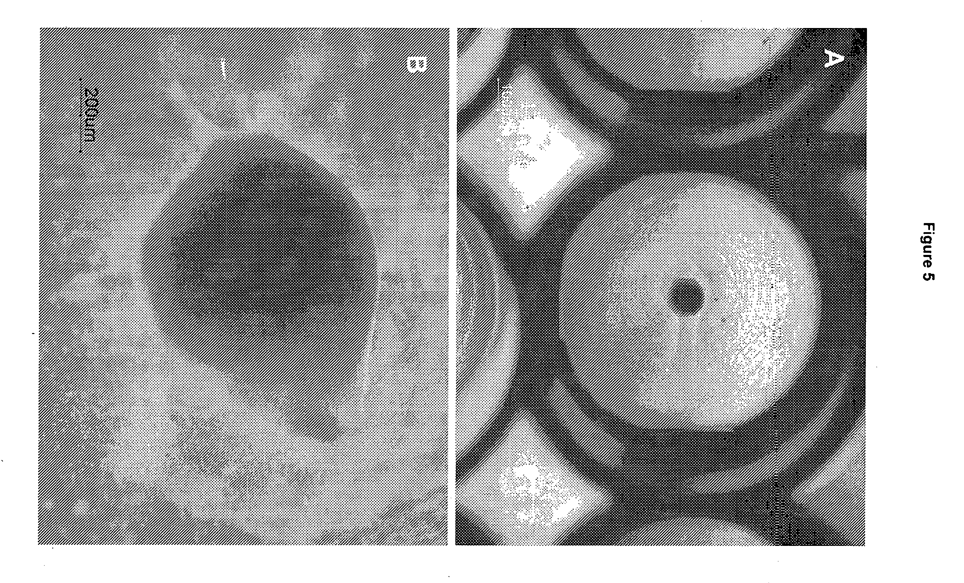

hES cells (SA002,5, p38) were dissociated into small cell clusters of between 50 and 100 cells per cluster and seeded into 96-well round-bottom polypropylene plates (Nunc, Kamstrupvej, Denmark) in 200µl cell culture medium containing Knock Out DMEM supplemented with 20% FBS, 1% penicillin-streptomycin, 1% Glutamax, 0.5 mmol/l β-mercaptoethanol and 1% non-essential amino acids (all from Invitrogen, Carlsbad, California) in a concentration of ca. 10000 cells per well. Following the plates were centrifugated at 400g for 5 minutes in a centrifuge (Eppendorf). The hES cells were let to differentiate for 30 days with medium change every second day. Photos were taken at day 12 of differentiation using a light microscope (Leica Microsystems AB, Sollentuna, Sweden). At this time point the cells have formed microtissue with a diameter of approximately 500 to 1000 µm. *(See* *figure 5**.)*

### Reference Example 3

### Formation of microtissues with contracting cardiomycytes without suspension EBs cultivation and plating steps

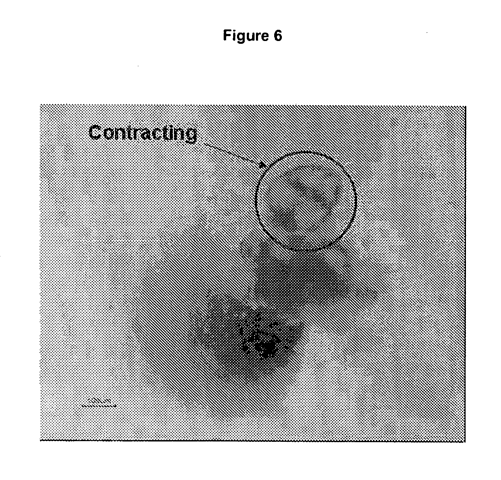

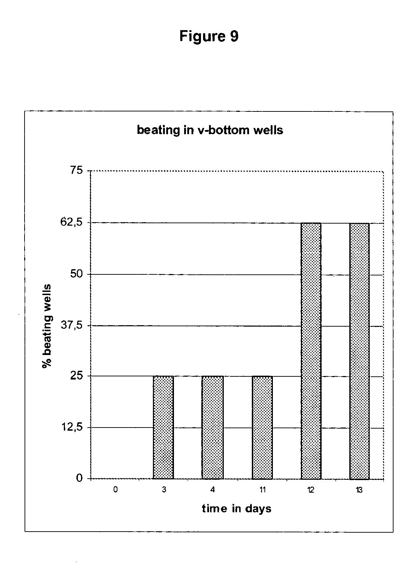

hES cells (SA002,5, p38) were dissociated into small cell clusters of between 50 and 100 cells per cluster and seeded into 96-well round-bottom plates (Nunc, Kamstrupvej, Denmark) in 200µl cell culture medium containing Knock Out DMEM supplemented with 20% FBS, 1% penicillin-streptomycin, 1% Glutamax, 0.5 mmol/I β-mercaptoethanol and 1% non-essential amino acids (all from Invitrogen, Carlsbad, California) in a concentration of ca. 10000 cells per well. Following, the plates were centrifugated at 400g for 5 minutes in a centrifuge (Eppendorf). Cell culture medium was changed every second to third day. The hES cells were let to differentiate for 12 days with medium change every second day. Photos were taken at day 12 of differentiation using a light microscope (Leica Microsystems AB, Sollentuna, Sweden). Rhythmically contracting areas appeared in approximately 30 % of the microtissues, indicating the presence of cardiomyocyte-like cells in the microtissues. (*See* *figure 6* *and* *figure 9**)*

### Reference Example 4:

### Formation of microtissues with all three germ layers present

hES cells (SA002) were dissociated into small cell aggregates and seeded into 96-well round-bottom Polypropylene plates (Nunc, Kamstrupvej, Denmark) in 200µl cell culture medium containing Knock Out DMEM supplemented with 20% FBS, 1% penicillin-streptomycin, 1% Glutamax, 0.5 mmol/l β-mercaptoethanol and 1% non-essential amino acids (all from Invitrogen, Carlsbad, California) in a concentration of ca. 10, 000 cells per well. Following the plates were centrifugated at 400g for 5 minutes in a centrifuge (Eppendorf). Cell culture medium was changed every second to third day. For proof-of-principle and to confirm that all germlayers were present in the formed microtissues these were analysed with RT-qPCR for the markers Oct-4 and hTert that are expressed in undifferentiated cells, class III beta-tubulin and nestin for ectoderm, alphafetoprotein for endoderm and desmin for mesoderm. The microtissues were differentiated for 20 days with medium change every second day. At day 20, the microtissues were collected in a microtube, washed twice with PBS (Invitrogen) and frozen as dry pellets at -80 °C (Thermo Forma -86 °C ULT freezer, Thermo Scientific, Basingstoke, United Kingdom). These samples were analyzed using RT-qPCR techniques by TATAA (Göteborg, Sweden). The following genes were tested: Oct-4 and hTert for early differentiation, class III beta-tubulin and sox-1 for ectodermal differentiation, AFP for endodermal and desmin for mesodermal differentiation. Results confirmed that all three germ layers were present, which makes the plates with the microtissues suitable for assay use for e.g. developmental toxicity using for instance a multi-well format QPCR.

Additionally, the time dependent expression of two to three genes per germ layer were analysed, i.e. Nkx2.5 and ACTC1 as mesodermal markers, AFP, HNF4-alpha and HGF as endodermal markers and Pax6 and MAP2 as ectodermal markers. hES cells of the cell line SA002 were differenitiated as microtissues in conical 96-well plates (Corning) in DMEM-F12 supplemented with 1% penicillin-streptomycin, 1% Glutamax, 0.5 mmol/l β-mercaptoethanol, 1% non-essential amino acids and 20% Knockout serum replacement. Samples were collected at day 0, 7 and 14. Experiments were performed in three independent runs.

RNA was isolated with an RNeasy Mini Kit (Qiagen, Hilden, Germany) according to the manufacturer's protocol. RNA concentration and protein contaminations were assessed spectrophotometrically (GeneQuantpro; GE Healthcare, Stockholm, Sweden). Reverse transcription was performed as follows. All concentrations are the final in the reverse transcriptase reaction (final volume was 20 µl). To ensure single mRNA strands, 500 ng RNA were incubated with 2.5 mM PCR Nucleotide Mix (Promega, Madison, USA)) and 12.5 µg/ml random primers (Promega) at 65°C for 5 min. Then 10 units/µl M-MLV reverse transcriptase (Promega) and 2 units/µl RNaseOUT (Invitrogen) was added with the respective M-MLV buffer and the samples were heated to 25°C for 10 min to allow annealing, 37oC for 1 hour for cDNA synthesis and 70°C for 15 min for inactivation of enzymes.

An AbiPrism 7500 sequence detector system in conjunction with TaqMan® Gene Expression Master Mix and TaqMan® Real-Time PCR Gene Expression Assays (all Applied Biosystems, Monza, Italy) was used for all genes according to the manufacturers protocol. Relative quantification was performed using the comparative CT method, based on housekeeping gene mRNA quantities. As house keeping genes 18S, UBC and CREBBP were used and gene expressions of target genes was normalised to the average of the two most stably expressed housekeeping genes.

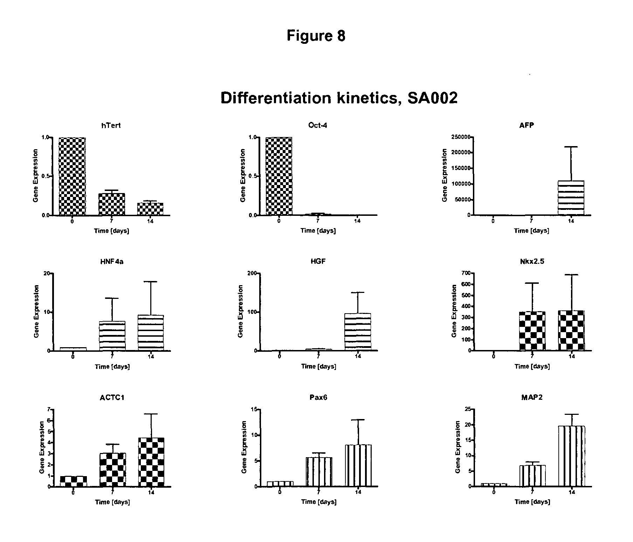

The results show that the differentiation of hES cells as microtissues in conical wells results in derivatives of the three germ layers. *(See* *figure 8**.)*

### Reference Example 5:

### Use of the culture system for performing a cytotoxicity assay on hES cells

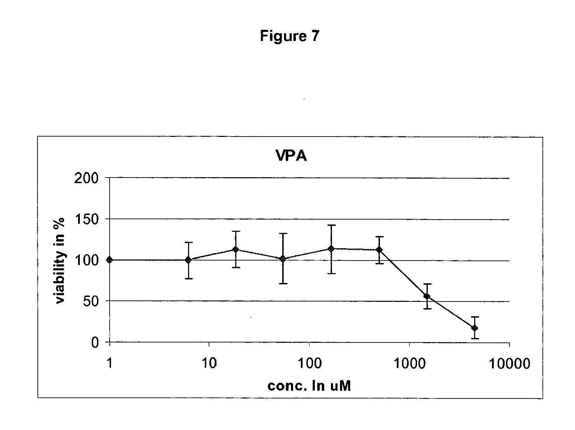

hES cells were dissociated into small cell clusters and seeded into 96-well plates (Nunc, Kamstrupvej, Denmark) in 100µl Test medium containing Knock Out DMEM supplemented with 20% Foetal bovine Serum (FBS), 1% penicillin-streptomycin, 1% Glutamax, 0.5 mmol/l β-mercaptoethanol and 1% non-essential amino acids (all Invitrogen, Carlsbad, California). Following, the plates were centrifugated for 5 minutes at 400g to assure attachment. After 24 hours a cytotoxicity test was started by adding 100µl toxicity solution to the test wells for incubation that had twice the concentration as the required end concentration (at day 0). Toxicity medium was changed on day 4 and 7 of the assay and on day 10 the plates were analysed measuring the reduction of Resazurin (Sigma, Stockholm, Sweden, CAS 62758-13-8) to the fluorescent Resofurin as described before (Evans *et al.,* 2001). Resofurin content was detected using a multi-detection reader (Fluostar Optima, BMG Labtech, Offenburg, Germany) measuring fluorescence, at the wave lengths 530 nm (excitation) and 590 nm (emission). The substance tested *(see* *figure 7*) was valproic acid (VPA), which is an anticonvulsant drug used to treat epilepsy and a well-known teratogen in all species including humans. (Sigma, CAS 99-66-1). It was dissolved in PBS at a concentration of 0.2M and stored at 4°C after aliquoting. It was tested in a 3-fold dilution series with the highest concentration being 4500 µM.

### Reference Example 6:

### Toxicity testing employing high content screening (HCS)

hES cells may be dissociated into small cell clusters or single cells and seeded into e.g. 0.1% gelatine (Sigma, Stockholm, Sweden) coated 96-well flat-bottom plates (Nunc, Kamstrupvej, Denmark) in 200µl cell culture medium, such as containing Knock Out DMEM supplemented with 20% (v/v) FBS, 1 %(v/v) penicillin-streptomycin, 1% (v/v) Glutamax, 0.5 mmol/l β-mercaptoethanol and 1% (v/v) non-essential amino acids (all from Invitrogen, Carlsbad, California) in a concentration of approximately 5.000 cells per well. Thereafter the plates may be centrifuged at e.g. 400g for roughly 5 minutes in a centrifuge (such as Heraeus, Thermo Scientific, Basingstoke, United Kingdom). After 24 hours the toxicity test may be started by adding 100µl toxicity solution to the test wells that had twice the concentration as the required end concentration (at day 0). Toxicity medium may then be changed throughout the assay, such as on day 4 and 7 of the assay and on for instance day 10 the plates may be prepared for analysis. Several toxic substance may be tested in suitable dilution series. DMSO may be used as one control substance and a suitable dilution range could then be a three-fold dilution series ranging from 1% to 0.01 %.

At the day of analysis (e.g. day 10) the plates may be fixed in for instance 4% paraformaldehyde/PBS for 20 minutes at room temperature and then washed twice 5 minutes with rinse buffer (TBST: 20mM Tris-HCL, pH 7.4, 0.15M NaCl, 0.05% Tween-20; Sigma). Next the cells may be permeabilised with e.g. 0.1% Triton X-100 (Sigma)/PBS (Gibco) for 10 minutes at room temperature and washed and subsequently treated with a blocking solution (such as 4% normal goat serum/PBS; Gibco). Primary antibodies directed against e.g. OCT-4 and hTert (Santa Cruz Biotechnology, Santa Cruz, CA, USA) may then be diluted to their final concentrations in the blocking solution and applied to the cells. In addition differentiation specific antibodies against the different germlayers, such as genes listed in table 1, may be used. After incubation and washing suitable secondary antibodies may be applied to the cells, exemplified by but not limited to FITC-, Cy3-, or TRITC-conjugated secondary antibodies (from e.g. Jackson ImmunoResearch Laboratories Inc., West Grove, PA, USA). The nuclei may be visualized by DAPI staining (Sigma Diagnostics, Stockholm, Sweden). Following, the plates may be analyzed using image analysis techniques, preferably an automated cell imager like the IN Cell Analyzer (IN Cell Analyzer 1000, GE Healthcare, Uppsala, Sweden) that comprises an automated Nikon™ microscope, high-resolution CCD camera, xenon lamp-based illumination, filter wheel based wavelength control, and laser based auto focus. Employing image analysis software packages, the instrument can acquire population-averaged and individual cell data from a wide variety of live-cell and fixed-cell applications.

*Table 1* List of marker genes for undifferentiated hES cells and for all three germ layers present in hES cell derived microtissue.

**Table 1**

| **Marker Genes:** | | | | |
|---|---|---|---|---|
| ***Undifferentiated hES cells*** | ***Differentiated hES cell derived microtissue*** | | | |
| | ***Ectoderm:*** | ***Endoderm:*** | ***Mesoderm:*** | ***Trophoblast:*** |
| Oct-4 | VIM | FN1 | COL1A1 | EOMES |
| hTert | CRABP2 | DCN | HAND1 | CDX2 |
| LIN28 | SEMA3A | H19 | IGF2 | |
| DNMT3B | NES | AFP | MSX1 | |
| LECT1 | TH | SERPINA1 | ACTC | |
| ZNF206 | TUBB3 | GATA4 | GATA6 | |
| GYLTL1B | PAX6 | | NKX2.5 | |
| LEFTY1 | GFAP | | | |
| LEFTY2 | | | | |
| GAL | | | | |
| UTF1 | | | | |
| NANOG | | | | |
| SOX2 | | | | |
| DUSP6 | | | | |

In parallel genetically engineered hES cells containing a marker gene, such as the GFP gene driven under a suitable promoter, such as the OCT-4 promoter may be seeded into e.g. a 0.1 % gelatine (Sigma, Stockholm, Sweden) coated 96-well flat-bottom plates (Nunc) in 200µl cell culture medium. These plates may then be treated with a substance in parallel with the wild-type hES cell plates following a procedure similar to that above but without performing any steps for immunohistochemistry. After 10 days the plates may be analyzed using an automated cell imager (IN Cell Analyzer 1000, GE Healthcare) and specific software that delivers quantitative data of the different fluorescent intensities and areas of expression. One big advantage with the latter approach, employing cells with reporter genes, is that a more homogeneous assay approach can be used, which means that washing or separation steps are not needed. In addition the reporter-gene approach has the advantage of enabling reading over time, i.e. not only giving a snap-shot of the cells at a specific moment (the moment of cell fixation).

Still one additional assay approach could be to use membrane binding antibodies to perform HCS immunohistochemistry on living cells. Such antibodies may be directly conjugated to a fluorescent group whereby several washing steps can be eliminated leading to a simplified and more homogeneous assay protocol. Further marker detection can be made by RT-qPCR using low density arrays.

### Reference Example 7:

### Automation of the culture system

The production and manipulation of hES cells is currently being scaled up using novel culture systems for bulk culture, therefore large amounts of hES cells will become available for the application in *in vitro* assays. The preparation, treatment and analysis of hES cells or cells derived from hES cells in multi-well assay plates according to our system and related method does not require manual selection or micromanipulation and can therefore be scaled up and automated using robotization. Suitable robots could be based on XYZ dispensing heads which allow pipetting to and from culture vessels such as liquid handling stations or could be based on a robotic arm which mimics the movements of a human being during culture vessel and pipette handling. Within the robotic system environmental parameters such as e.g. temperature, nutrient supply, pH, pressure, shear forces and oxygen should be maintained within optimal limits.

### Reference Example 8:

### Adjusted enzymatic passage system for use in differentiation and assay systems

hESCs were expanded in a culture system consisting of high density HFF feeder cells and VitroHESTM medium supplemented with 4 ng/ml hrbFGF. After enzymatic dissociation for 4-6 minutes at 37oC in 1X TrypLE™ (Invitrogen) or 1X Trypsin/EDTA (Invitrogen), single cells were seeded onto high density HFF feeders (70 000 HFF per cm2) at routine split ratios between 1:4 and 1:40. Culture medium was replaced with fresh VitroHESTM + 4 ng/ml hrbFGF every 2-3 days. Depending on the growth speed of the individual hESC line the cells were passaged every 6-12 days. The single cell suspension were in the final or last steps before the use in the differentiation and assay system seeded onto feeder cells, wherein the feeder cells are present at a low density such as below about 50,000 cells/cm², about 10,000 cells/cm², about 15,000 cells/cm², about 20,000 cells/cm², about 25,000 cells/cm², about 30,000 cells/cm², about 35,000 cells/cm² ,about 40,000 cells/cm².This step considerably facilitates the removal of hES cells from the feeder and decreases the number of hFF cells needed for use in differentiation applications for toxicity testing or in systems for derivation differentiated cells types from hES cell lines. This allows a straightforward handling of hES cell for introduction to the present differentiation and assay system allowing up scaling and automation.

### Reference Example 9

### Screening assay for compounds to induce bone, cartilage and fat differentiation

hES cell derived mesenchymal progenitor cells are dissociated to a single cell suspension using TrypLE Select, resuspended in DMEM/F12 + 10% FBS + 1% P/S and seeded into conical well polypropylene 96 well plates at 10000 cells/well. The plates are centrifuged at 400 g for 10 min to aggregate the cells. The plates are incubated for 7 days at 37 degrees in a humidified atmosphere and 5% CO₂ to allow the formation of mesodermal microtissues. The microtissues are then used directly in the 96 well plate for a screening assay for novel chemical entities (NCEs) to induce bone, cartilage and fat differentiation.

### Reference Example 10

### Screeing cardiomyocyte microtissues for cardiotoxicants

hES cell derived cardiomyocytes are dissociated to a single cell suspension using TrypLE Select, for selection and purification. The purified cardiomyocytes are resuspended in DMEM/F12 + 10% FBS + 1% P/S and seeded into conical well polystyrene 96 well plates at 20000 cells/well. The plates are centrifuged at 400 g for 10 min to aggregate the cells. The plates containing the cardiomyocyte microtissues are incubated for 4 days at 37 degrees in a humidified atmosphere and 5% CO₂. The cardiomyocyte microtissues are then used directly in the 96 well plate for a screening assay for cardiotoxicants.

## Claims

1. A combined differentiation and assay method for human embryonic stem (hES) cells, wherein the combined method is performed in the very same plate without any transfer of the cells, the method comprising the steps:
(i) dissociation of the hES cells into single cells, clusters or a mixture thereof;
(ii) seeding of the dissociated hES cells into one or more wells of one or more multi-well format plates;
(iii) application of a centrifugal force to the seeded hES cells for enhanced coalescence; and
(iv) differentiating the hES cells into progenitor cells or fully differentiated cells of the endodermal lineage and assaying the cells without replating;
wherein no replating of cells takes place in steps (ii) to (iv).

2. A combined differentiation and assay method according to claim 1, wherein the hES-derived cells are a mixed population of more than one cell type.

3. A combined differentiation and assay method according to claim 1, wherein the hES-derived cells are an essentially pure population.

4. A combined differentiation and assay method according to any of the above claims where one or more culture media for incubation of the cells contains a serum component, selected from a group including FBS, serum replacement, human serum, albumin, insulin, and transferin, at a concentration of 0.5-50%, such as 5-30% or such as 10-20%.

5. A combined differentiation and assay method according to the above claims comprising:
i) dissociating the hES cells with one or more dissociation agents;
ii) seeding of the hES cells into one or more individual wells in one or more multi-well format plates at a density of 1000 -1000 000 cells per well;
iii) applying a centrifugal force, such as 100-1000 G, for enhanced coalescence of the hES cells;
iv) incubating the hES cells in one or more culture media containing a serum component, selected from a group including, but not limited to, FBS, serum replacement, human serum, albumin, insulin, and transferin, at a concentration of such as 5-30%, to differentiate them into progenitor cells or fully differentiated cells of the endodermal lineage and assaying the cells without replating.

6. The combined differentiation and assay method according to claim 1, where a survival promoting substance is added during step (i) and/or (ii) or wherein the cells are strained in step (ii).

7. The combined differentiation and assay method according to claim 1, for use in drug discovery and/or for safety assessment and toxicity studies.

8. The combined differentiation and assay method according to claim 1 for preparing cells for detecting or measuring cell differentiation, wherein the differentiation is measured by measuring gene or protein levels.

9. A method according to any of claims 1-8, for running an assay based on genetically engineered cells.

10. Use of a kit in a method according to any of claims 1-9, the kit comprises one or more dissociation agents selected from Trypsin, Trypsin/EDTA, Collagenase IV and Dispase.

11. Use of a kit according to claim 10, the kit further comprises positive control substances, negative control substances or additional cell types for use as control populations.

12. Use of a kit according to claim 10 or 11, the kit further comprising one or more types of multi-well plates.

## Patentansprüche

1. Kombiniertes Differenzierungs- und Assayverfahren für humane embryonale Stammzellen (hES), wobei das kombinierte Verfahren in genau derselben Platte ohne Übertragung der Zellen durchgeführt wird, welches Verfahren die folgenden Schritte umfasst:
(i) Dissoziation der hES-Zellen in einzelne Zellen, Cluster oder eine Mischung davon;
(ii) Aussähen der dissoziierten hES-Zellen in einen oder mehrere Well(s) einer oder mehrerer Multiwell-Platte(n);
(iii) Ausüben einer Zentrifugalkraft auf die ausgesähten hES-Zellen zur erhöhten Koaleszenz; und
(iv) Differenzieren der hES-Zellen zu Progenitorzellen oder völlig differenziierten Zellen endodermaler Abstammung und Analysieren der Zellen ohne Neuplattieren;
wobei kein Neuplattieren von Zellen in den Schritten (ii) bis (iv) erfolgt.

2. Kombiniertes Differenzierungs- und Assayverfahren nach Anspruch 1, wobei die hES-abgeleiteten Zellen eine Population aus mehr als einem Zelltypen sind.

3. Kombiniertes Differenzierungs- und Assayverfahren nach Anspruch 1, wobei die hES-abgeleiteten Zellen eine im Wesentlichen reine Population sind.

4. Kombiniertes Differenzierungs- und Assayverfahren nach einem der obigen Ansprüche, wobei eines oder mehrere Kulturmedien für die Inkubation der Zellen einen Serumbestandteil enthält/enthalten, welcher aus einer Gruppe umfassend FBS, Serumersatz, humanes Serum, Albumin, Insulin und Transferin, bei einer Konzentration von 0,5-50 %, wie beispielsweise 5-30 % oder beispielsweise 10-20 %, ausgewählt ist.

5. Kombiniertes Differenzierungs- und Assayverfahren nach den vorgehenden Ansprüchen, umfassend:
i) Dissoziieren der hES-Zellen mit einem oder mehreren Dissoziationsmittel(n);
(ii) Aussähen der hES-Zellen in einen oder mehrere einzelne(n) Well(s) in einer oder mehrerer Multiwell-Platte(n) bei einer Dichte von 1.000-1.000.000 Zellen je Well;
iii) Ausüben einer Zentrifugalkraft, wie beispielsweise 100-1000 G, zur erhöhten Koaleszenz der hES-Zellen;
iv) Inkubieren der hES-Zellen in einem oder mehreren Kulturmedien umfassend einen Serumbestandteil, welcher aus einer Gruppe umfassend, jedoch nicht darauf beschränkt, FBS, Serumersatz, humanes Serum, Albumin, Insulin und Transferin, bei einer Konzentration von beispielsweise 5-30 %, ausgewählt ist, um sie zu Progenitorzellen oder völlig differenziierten Zellen endodermaler Abstammung zu differenziieren und die Zellen ohne Neuplattieren zu analysieren.

6. Kombiniertes Differenzierungs- und Assayverfahren nach Anspruch 1, wobei eine das Überleben begünstigende Substanz in dem Schritt (i) und/oder (ii) zugegeben wird, oder wobei die Zellen in dem Schritt (ii) erschöpft werden.

7. Kombiniertes Differenzierungs- und Assayverfahren nach Anspruch 1, zur Verwendung für die Arzneimittelforschung und/oder für die Sicherheitsbewertung und Studien der Toxizität.

8. Kombiniertes Differenzierungs- und Assayverfahren nach Anspruch 1 zur Vorbereitung von Zellen für das Detektieren oder Messen von Zellendifferenzierung, wobei die Differenzierung durch das Messen von Gen- oder Proteinwerten ermittelt wird.

9. Verfahren nach einem der Ansprüche 1 bis 8 zum Ablaufen eines Assays, welcher auf genetisch veränderten Zellen basiert.

10. Verwendung eines Kits bei einem Verfahren nach einem der Ansprüche 1 bis 9, welches Kit ein oder mehrere Dissoziationsmittel umfasst, welche(s) aus Trypsin, Trypsin/EDTA, Kollagenase IV und Dispase ausgewählt ist/sind.

11. Verwendung eines Kits nach Anspruch 10, welches Kit zusätzlich Positivkontrollsubstanzen, Negativkontrollsubstanzen oder zusätzliche Zelltypen zur Verwendung als Kontrollpopulationen umfasst.

12. Verwendung eines Kits nach Anspruch 10 oder 11, welches Kit ferner eine oder mehrere Art(en) von Multiwell-Platten umfasst.

## Revendications

1. Procédé combiné de différenciation et d'essai pour des cellules souches embryonnaires (hES), le procédé combiné étant réalisé dans tout à fait la même plaque sans aucun transfert des cellules, le procédé comprenant les étapes de:
(i) la dissociation des cellules hES dans des cellules individuelles, des clusters ou un mélange de ceux-ci;
(ii) l'ensemencement des cellules hES dissociées dans un ou plusieurs puits ou une ou plusieurs plaques de format multipuits;
(iii) l'application d'une force centrifuge aux cellules hES ensemencées pour améliorer la coalescence; et
(iv) la différenciation des cellules hES dans des cellules progénitrices ou des cellules totalement différenciées du lignage endodermique et l'essai des cellules sans préparation d'elles en plaques;
aucune préparation de cellules en plaques n'ayant lieu dans les étapes de (ii) à (iv).

2. Procédé combiné de différenciation et d'essai selon la revendication 1, dans lequel les cellules hES dérivées sont une population mixte de plus d'un type de cellule.

3. Procédé combiné de différenciation et d'essai selon la revendication 1, dans lequel les cellules hES dérivées sont une population essentiellement pure.

4. Procédé combiné de différenciation et d'essai selon l'une quelconque des revendications précédentes, dans lequel un ou plusieurs milieux de culture pour l'incubation des cellules contiennent un composant de sérum, choisi dans un groupe comprenant FBS, le remplacement du sérum, le sérum humain, l'albumine, l'insuline et la transferrine, à une concentration comprise entre 0,5 et 50%, par exemple entre 5 et 30%, ou par exemple entre 10 et 20%.

5. Procédé combiné de différenciation et d'essai selon l'une quelconque des revendications précédentes, comprenant:
i) la dissociation des cellules hES avec un ou plusieurs agents de dissociation;
ii) l'ensemencement des cellules hES dans un ou plusieurs puits individuels ou une ou plusieurs plaques de format multipuits à une densité comprise entre 1000 et 1000 000 cellules par puits;
(iii) l'application d'une force centrifuge, par exemple comprise entre 100 et 1000 G, pour améliorer la coalescence des cellules hES;
iv) l'incubation des cellules hES dans un ou plusieurs milieux de culture contenant un composant de sérum, choisi dans un groupe comprenant, mais sans s'y limiter, FBS, le remplacement du sérum, le sérum humain, l'albumine, l'insuline et la transferrine, à une concentration comprise entre 5 et 30%, pour les différencier dans des cellules progénitrices ou des cellules totalement différenciées du lignage endodermique et l'essai des cellules sans préparation d'elles en plaques.

6. Procédé combiné de différenciation et d'essai selon la revendication 1, dans lequel une substance promotrice de survie est ajoutée lors de l'étape (i) et/ou (ii) ou dans lequel les cellules sont contraintes dans l'étape (ii).

7. Procédé combiné de différenciation et d'essai selon la revendication 1, pour une utilisation dans la découverte de médicaments et/ou pour une évaluation de la sécurité et de toxicité.

8. Procédé combiné de différenciation et d'essai selon la revendication 1, pour la préparation de cellules pour la détection ou la mesure de la différenciation cellulaire, la différenciation étant mesurée en mesurant les niveaux de gène ou de protéine.

9. Procédé selon l'une quelconque des revendications 1 à 8, pour l'exécution d'un essai basé sur des cellules génétiquement modifiées.

10. Utilisation d'un kit dans un procédé selon l'une quelconque des revendications 1 à 9, le kit comprenant un ou plusieurs agents de dissociation, choisis parmi la trypsine, la trypsine/EDTA, la collagénase IV et la dispase.

11. Utilisation d'un kit selon la revendication 10, le kit comprenant en outre des substances de contrôle positives, des substances de contrôle négatives ou des types de cellules supplémentaires pour une utilisation en tant que populations de contrôle.

12. Utilisation d'un kit selon la revendication 10 ou 11, le kit comprenant en outre un ou plusieurs types de plaques multipuits.
